# EUROPEAN PATENT APPLICATION

(11) **EP 4 027 295 A1**
(43) Date of publication of application: **13.07.2022**
(21) Application number: 20859943.1
(22) Date of filing: 19.08.2020
(51) Int. Cl.: G06Q 50/00, G06N 20/00

(54) **MATERIAL PROPERTY PREDICTION DEVICE AND MATERIAL PROPERTY PREDICTION METHOD**

(30) Priority: 03.09.2019 JP 2019160261
(71) Applicant: Hitachi, Ltd., Tokyo 100-8280 (JP)
(72) Inventor: KANAZAWA, Takuya, Tokyo 100-8280 (JP); ASAHARA, Akinori, Tokyo 100-8280 (JP); HAYASHI, Takayuki, Tokyo 100-8280 (JP); MORITA, Hidekazu, Tokyo 100-8280 (JP)
(74) Representative: Mewburn Ellis LLP
(86) International application number: PCT/JP2020/031263
(87) International publication number: WO 2021/044846

(57) **Abstract**

The present invention efficiently generates effective compound feature quantities reflecting expert knowledge, and thereby accurately predicts physical properties of an unknown compound.

A device for predicting a material property using a case-by-case material database storing a plurality of case databases. The case databases include a plurality of records that record structural information about material structures in association with material properties about properties of materials. This device is characterized in including: a chemical space designation unit that receives a designation of at least one case database; an autoencoder learning unit that generates an autoencoder for converting structural information corresponding to the case database received by the chemical space designation unit to multi-variables; and a material property prediction unit that predicts material properties using the multi-variables converted by the autoencoder generated by the autoencoder learning unit.

## Description

### TECHNICAL FIELD

The present invention relates to a machine learning system for predicting physical properties of a material.

### BACKGROUND ART

In the related art, in order to find out physical properties of a compound, it was necessary to actually generate the compound by a synthesis experiment and perform measurement directly by an experiment. However, in these days, as a large amount of compound physical properties data is accumulated, an attempt has also been performed in which physical properties with respect to an unknown compound are predicted by processing the data with a machine learning algorithm.

Since there are enormous combinations of unknown compounds, it is expected that the efficiency of material development can be greatly improved insofar as a substance group having desired properties can be selected without performing such an experiment. In order to attain such an improvement, it is necessary to increase the reliability of the prediction using machine learning. However, in the present conditions, since there are no sufficient methods for converting the structure of the compound to manageable feature quantities (a descriptor) that are suitable for the machine learning, a compound physical property prediction system having high reliability has not been attained yet.

For example, in Non-Patent Document 1, a method is disclosed in which structural information of a compound is converted to a multi-variable vector using a known variational autoencoder to be used in physical property prediction.

In addition, in Patent Document 1, a method is disclosed in which a molecular structure of an organic compound is represented by a plurality of types of fingerprint methods to be used in physical property prediction. In Patent Document 2, a machine learning system for drug design is disclosed in which information of a compound is encoded as a latent variable by a known autoencoder.

### CITATION LIST

### PATENT DOCUMENT

Patent Document 1: WO 2019/048965 A1
Patent Document 2: JP 2019-502988 A

### NON-PATENT DOCUMENT

Non-Patent Document 1: R. Gomez-Bombarelli, J. N. Wei, D. Duvenaud, J. M. Hernandez-Lobato, B. Sanchez-Lengeling, D. Sheberla, J. Aguilera-Iparraguirre, T. D. Hirzel, R. P. Adams, A. Aspuru-Guzik, "Automatic Chemical Design Using a Data-Driven Continuous Representation of Molecules", ACS Cent. Sci. 2018, 4, 268-276.

### SUMMARY OF THE INVENTION

### PROBLEMS TO BE SOLVED BY THE INVENTION

In a situation where a substance having a specific physical property is required to be searched by a machine learning method, it is required to generate necessary and sufficient feature quantities (a descriptor) from a structural formula of the compound in order to effectively express the physical property such that a computer accurately understands the physical property.

However, in the case of generating the feature quantities from the structural formula by the method of the related art, for example, free software such as RD Kit, a set of versatile and general feature quantities are generated, which may be redundant for a desired analysis purpose. In order to select only feature quantities important for the prediction of desired physical properties from the redundant feature quantities of the compound, it is desired to prepare a sufficient amount of training data (a pair of a compound and a teacher label (a desired physical property value)), train a prediction model on the basis of the training data, and extract feature quantities determined to be useful by the prediction model. However, it is difficult to execute such a process in a situation where the training data is not sufficiently obtained.

In addition, when using machine learning means capable of converting a structural formula to a continuous multi-variable vector, such as a known autoencoder, it is general to use a large-scale open database of a compound, such as ChEMBL, in the learning of a model (a neural network or the like), and such a data set includes many substances in addition to a substance having a specific physical property, and thus, the continuous multi-variable vector generated from the large-scale open database is not necessarily optimal for a desired analysis purpose.

The present invention has been made in consideration of the problems described above, and an object thereof is to accurately predict physical properties of an unknown compound by enabling effective compound feature quantities (an explanatory variable) reflecting expert knowledge to be efficiently generated.

### SOLUTIONS TO PROBLEMS

One preferred aspect of the present invention is a device for predicting a material property using a case-by-case material database storing a plurality of case databases. The case database includes a plurality of records recording structural information about material structures in association with material properties about properties of materials. The device includes chemical space designation unit receiving a designation of at least one case database, an autoencoder learning unit generating an autoencoder for converting structural information corresponding to the case database received by the chemical space designation unit to multi-variables, and a material property prediction unit predicting material properties using the multi-variables converted by the autoencoder generated by the autoencoder learning unit.

Another preferred aspect of the present invention is a material property prediction method executing a first step of preparing a first database including a plurality of records recording structural information about material structures, a second step of extracting structural information from the first database prepared in the first step, a third step of training an autoencoder for converting structural information to multi-variables using the structural information extracted in the second step, a fourth step of preparing a second database including a plurality of records recording structural information about material structures in association with material properties about properties of materials, a fifth step of extracting structural information from the second database prepared in the fourth step, a sixth step of converting the structural information extracted in the fifth step to multi-variables using the autoencoder, a seventh step of obtaining explanatory variables on the basis of the multi-variables converted in the sixth step and obtaining objective variables on the basis of material properties extracted from the second database, and an eighth step of generating a prediction model for assuming the objective variables from the explanatory variables using the explanatory variables and the objective variables.

### EFFECTS OF THE INVENTION

According to the present invention, it is possible to efficiently generate effective compound feature quantities reflecting expert knowledge, and thereby accurately predict physical properties of an unknown compound.

### BRIEF DESCRIPTION OF THE DRAWINGS

Fig. 1 is a block diagram illustrating a functional configuration of a material property prediction device in Example.
Fig. 2 is a flowchart of processing of the material property prediction device in Example.
Fig. 3 is an image of an example of a display screen of an experimental data receiving unit in Example.
Fig. 4 is a table of an example of a data structure of a case-by-case material database in Example.
Fig. 5 is an image of an example of a display screen of a chemical space designation unit in Example.
Fig. 6 is a table of an example of a data structure of output to an autoencoder learning unit from the case-by-case material database in Example.
Fig. 7 is a conceptual diagram illustrating a configuration of an autoencoder in Example.
Fig. 8 is an image of an example of a display screen of a material property prediction receiving unit in Example.
Fig. 9 is a table of an example of a data structure of input to the material property prediction receiving unit in Example.
Fig. 10 is a flowchart of processing of a material property prediction unit in Example.
Fig. 11 is an image of an example of a display screen of a display unit in Example.
Fig. 12 is a conceptual diagram illustrating a usage image in Example.

### MODE FOR CARRYING OUT THE INVENTION

Hereinafter, Example of the present invention will be described in detail with reference to the drawings. However, the present invention is not construed as being limited to the contents described in the following embodiments. It is easily understood by a person skilled in the art that a specific configuration can be changed within a range not departing from the idea or gist of the present invention.

In the configuration of the present invention described below, the same reference numerals will be used in common for the same parts or parts having similar functions in different drawings, and the repeated description may be omitted.

In a case where there are a plurality of elements having the same or similar functions, the elements may be described by applying different subscripts to the same reference numerals. However, in a case where it is not necessary to distinguish the plurality of elements, the elements may be described without the subscripts.

Herein, the representations such as "first", "second", and "third" are applied to identify constituents, and do not necessarily limit the number, the order, or the content thereof. In addition, the numbers for identifying the constituents are used for each context, and the numbers used in one context do not necessarily indicate the same configuration in the other contexts. In addition, a constituent identified by a certain number may have the function of a constituent identified by the other number.

The position, the size, the shape, the range, and the like of each configuration illustrated in the drawings may not represent the actual position, size, shape, range, and the like, in order to facilitate understanding of the present invention. Accordingly, the present invention is not necessarily limited to the position, the size, the shape, the range, and the like illustrated in the drawings.

A part of the description herein is configured by the publications, patents, and patent applications cited herein.

Herein, constituents represented in the singular form include the plural form, unless explicitly stated in the context.

### [Example 1]

One aspect of Example to be described below is a device for predicting a material property, and the device includes a case-by-case material database recording a structural formula of a material, an experiment condition, and a material property in association with each other case by case, a chemical space designation unit receiving a designation of a single case or a plurality of cases from a user, an autoencoder learning unit having a function of generating an autoencoder that is a model having a property of enabling a structural formula to be restored from multi-variables after converting the structural formula to the multi-variables, with respect to a set of structural formulas corresponding to the cases received by the chemical space designation unit, and a material property prediction unit predicting material properties by generating explanatory variables using the autoencoder generated by the autoencoder learning unit.

According to this example, even in a situation where there is no sufficient amount of compound training data, it is possible to provide an analysis system that is capable of generating effective feature quantities incorporating expert knowledge, and thereby predicting physical properties with a high accuracy.

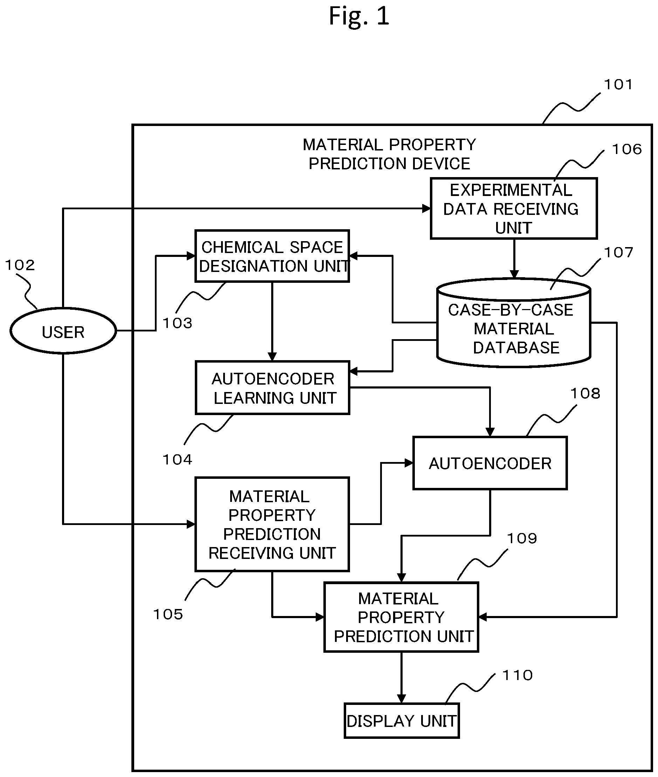

Fig. 1 is a functional configuration block diagram illustrating an example of a functional configuration of a material property prediction device in this example. In Fig. 1, a material property prediction device 101 includes an experimental data receiving unit 106 receiving material experimental data from a user 102, a case-by-case material database 107 for accumulating the material experimental data case by case, a chemical space designation unit 103 receiving a designation of a chemical space from the user 102, an autoencoder learning unit 104 executing the preparation and the learning of an autoencoder, an autoencoder 108, a material property prediction receiving unit 105 receiving a material list of a prediction target from the user 102, a material property prediction unit 109 predicting material properties, and a display unit 110 displaying a prediction result to the user 102.

Note that, the material property prediction device 101 is attained by a device including a processor, a memory, a storage device, and a communication unit (an interface), which is a general information processing device, as hardware. That is, the experimental data receiving unit 106, the chemical space designation unit 103, and the material property prediction receiving unit 105 receive data input by the communication unit, and the case-by-case material database 107 stores data in the storage device. In addition, the autoencoder learning unit 104, the autoencoder 108, and the material property prediction unit 109 are executed by software processing in which a program stored in the memory is executed by a processor. In addition, the display unit 110 functions as both of an operating unit and a display unit, which are generally used, for example, includes a display, a keyboard, a mouse, and the like, and may be a display including a touch panel.

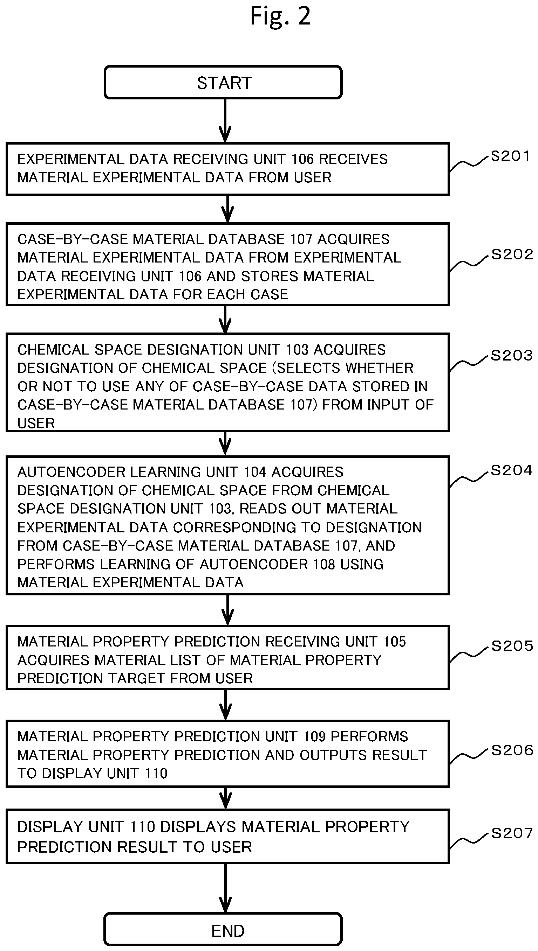

Fig. 2 is a flowchart of the processing of the material property prediction device 101 in this example. In Fig. 2, in step S201, the experimental data receiving unit 106 receives the material experimental data from the user 102.

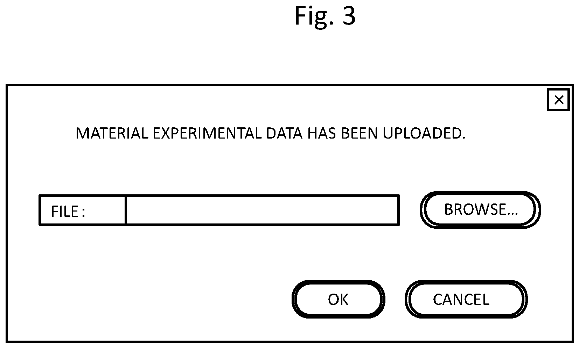

In Fig. 3, an example of an input receiving screen of the experimental data receiving unit 106. In a typical example, the material experimental data is already stored in a storage medium or the like, as an electronic file. As illustrated in Fig. 3, the user designates a file name of the material experimental data using a mouse, a keyboard, or the like, uploads the input, and confirms the input by pressing an OK button. In addition, the input can be corrected by a cancel button.

Returning to Fig. 2, in step S202, the case-by-case material database 107 acquires the material experimental data from the experimental data receiving unit 106 and stores the material experimental data for each of the cases.

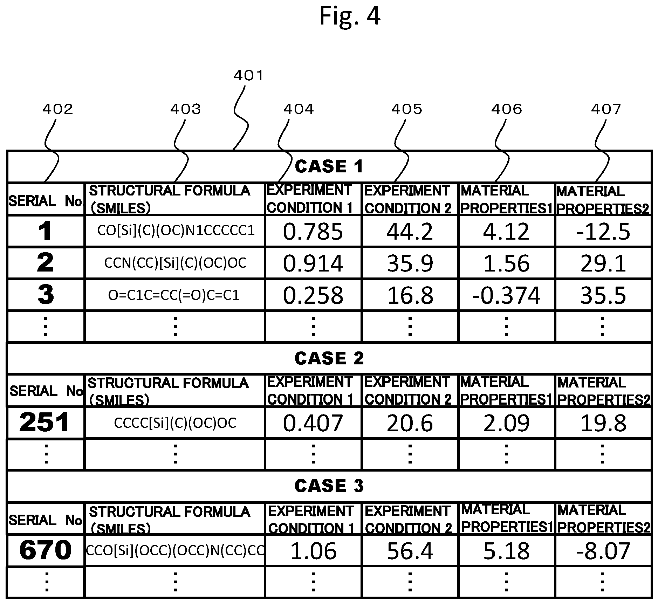

Fig. 4 illustrates the format of the data stored in the case-by-case material database 107. As illustrated in Fig. 4, the data includes a case number 401, a serial number 402, a structural formula 403 of a compound, experiment conditions 404 and 405, and material property values 406 and 407. The structural formula of the compound can be simply represented by using a simplified molecular-input line-entry system (SMILES) format, but is not necessarily limited thereto, and for example, a molecular structure may be treated as data in a graph format. In addition, the experiment condition may not be included in the data, and there may be a plurality of material property values or a single material property value.

As illustrated in Fig. 4, the case-by-case material database 107 is stored separately for each case data item (case database). In this example, one case data item includes a plurality of records in which the experiment conditions 404 and 405 and the material property values 406 and 407 are unified by data having the same definition or type. In this example, one record corresponds to one material structure.

Each of the case data items is data in which at least one of a target material, the definition of material properties, a preparation subject of the material, a preparation purpose of the material, a preparation time of the material, a preparation facility of the material, and the like is different, and for example, is data of an experiment result relevant to different themes. Accordingly, the definition or type of material structures, production·experiment conditions, and material properties may be different for each of the cases. Appendant information such as a preparation subject, a preparation purpose, a preparation time, a preparation facility, and a theme of data, for example, may be stored in association with the case data, as text information to be capable of being referred to or searched by the user. In this case, the chemical space designation unit 103 includes a graphical user interface (GUI) for searching the case data with a keyword or the like. The user is capable of extracting the case data to be used by using a search function.

Returning to Fig. 2, in step S203, the chemical space designation unit 103 acquires the designation of the chemical space from the input of the user 102.

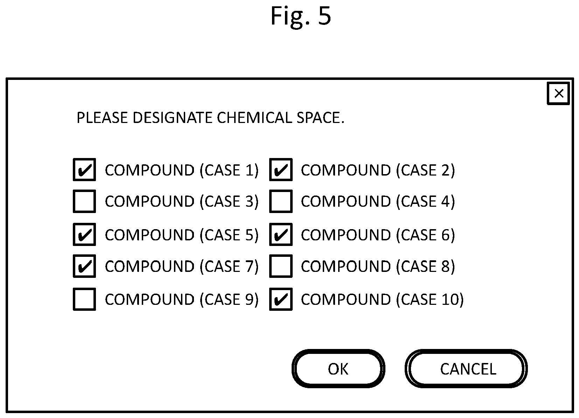

Fig. 5 illustrates an example of an input receiving screen of the chemical space designation unit 103. As illustrated in Fig. 5, the user is capable of selecting the compound case data to be used from the case-by-case material database 107.

Returning to Fig. 2, in step S204, the autoencoder learning unit 104 acquires the designation of the chemical space from the chemical space designation unit 103, reads out the material experimental data corresponding to the designation from the case-by-case material database 107, and performs the learning of the autoencoder 108 using the material experimental data.

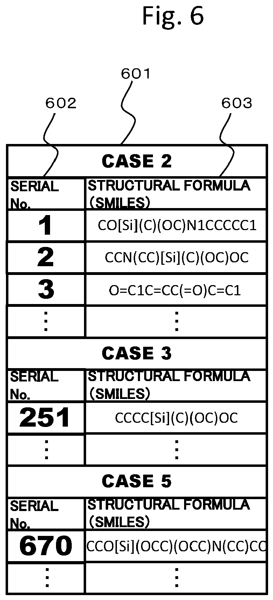

Fig. 6 is the structure of the data that is received by the autoencoder learning unit 104 from the case-by-case material database 107. The data includes a case number 601, a serial number 602 for designating the compound, and a structural formula 603 of the compound.

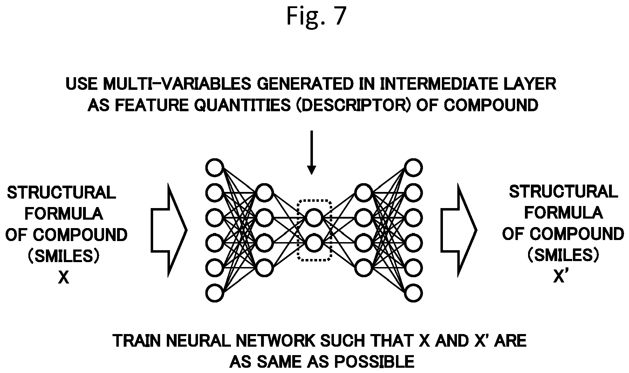

Fig. 7 illustrates the configuration and a learning method of the autoencoder 108. The autoencoder is a known dimension reduction machine using a neural network. In the autoencoder, a neural network referred to as an encoder lowers the dimension of high-dimensional input information, and another neural network referred to as a decoder, which receives the information, restores the received information to perform output close to the initial high-dimensional input. Such neural networks are trained in order to minimize an error in the input and output.

In a case where the structural formula is input and output as character information such as SMILES, a recurrent (recursive) neural network is suitable as an encoder and a decoder. In this example, the autoencoder is trained such that the autoencoder learning unit 104 converts structural formula information of the compound read out from the case-by-case material database 107 to a low-dimensional numerical vector. Note that, in this example, the autoencoder is used, but a known variational autoencoder may be used instead. In addition, the configuration of the autoencoder illustrated in Fig. 7 is an example, and an internal structure (the number of layers or the number of units) of the neural network is not limited to that illustrated in Fig. 7.

Returning to Fig. 2, in step S205, the material property prediction receiving unit 105 acquires a material list of a material property prediction target from the user 102.

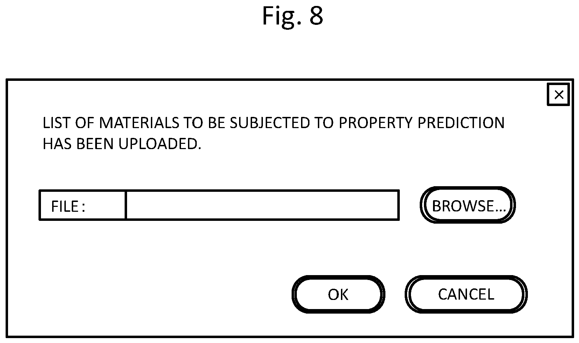

Fig. 8 is an example of an input receiving screen of the material property prediction receiving unit 105. The user is capable of uploading the material list by a mouse operation and a keyboard operation, confirming the input by pressing the OK button, and correcting the input by pressing the cancel button.

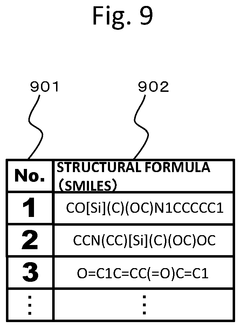

Fig. 9 is a data structure of the material list that is received by the material property prediction receiving unit 105. As illustrated in Fig. 9, the data includes a number 901 and structural formula information 902 of the compound.

Returning to Fig. 2, in step S206, the material property prediction unit 109 performs material property prediction and outputs a prediction result to the display unit 110.

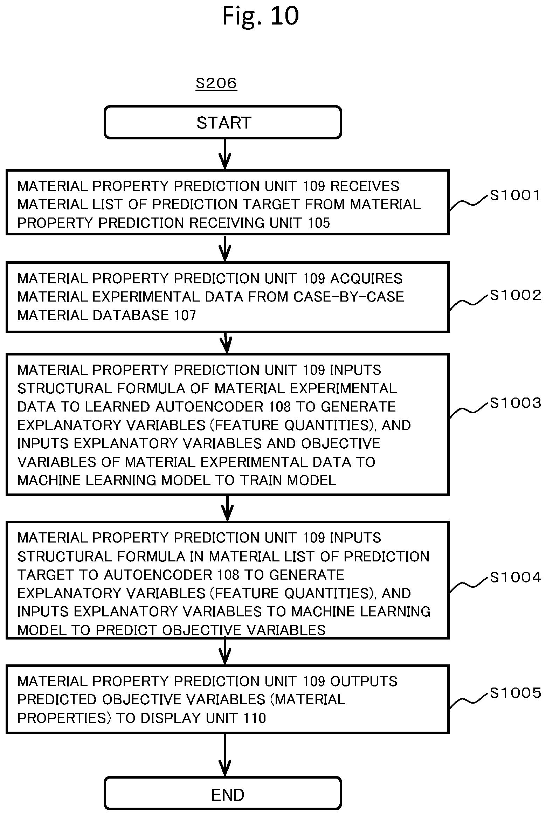

Fig. 10 is a flowchart of processing that is performed by the material property prediction unit 109 in step S206. In Fig. 10, first, in step S1001, the material property prediction unit 109 receives the material list of the prediction target of the structure illustrated in Fig. 9 from the material property prediction receiving unit 105.

In step S1002, the material property prediction unit 109 acquires the material experimental data from the case-by-case material database 107. The structure of the material experimental data is illustrated in Fig. 4.

In step S1003, the material property prediction unit 109 inputs the structural formula information of the material experimental data to the learned autoencoder 108 to generate feature quantities (a descriptor) of the compound. Then, the material property prediction unit 109 inputs the feature quantities and the material property value (objective variables) of the material experimental data to a machine learning model train a prediction model.

In the machine learning, for example, an arbitrary machine learning algorithm such as linear regression, a decision tree, a support vector machine, a neural network, a random forest, Gaussian process regression, gradient boosting, logistic regression, and a k-nearest neighbor algorithm can be used.

In step S1004, first, the material property prediction unit 109 inputs the structural formula in the material list of the prediction target to the autoencoder 108 to generate the feature quantities (the descriptor), and then, inputs the feature quantities to the prediction model trained in previous step S1003 to predict the material property value with respect to the prediction target material. In step S1005, the material property prediction unit 109 outputs a material property prediction value output by the prediction model to the display unit 110.

Returning to Fig. 2, in step S207, the display unit 110 displays a material property prediction result to the user 102.

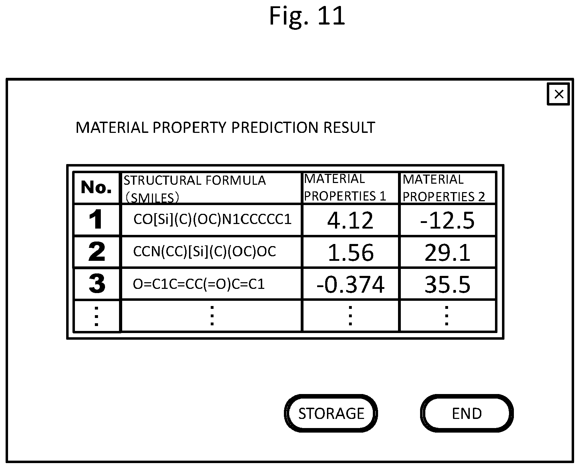

Fig. 11 illustrates an example of a result display screen of the display unit 110. Here, a list of the compound of the prediction target and the material property prediction value thereof is displayed, and the user is capable of storing the result by a storage button and ending the display by an end button.

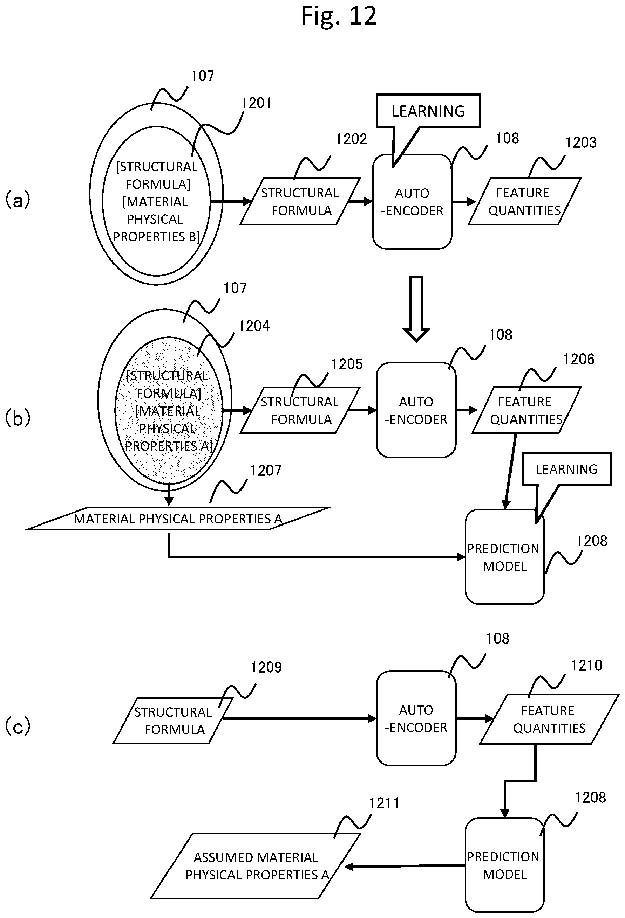

Fig. 12 is a conceptual diagram illustrating a usage image of this example. One characteristic of this example is that in the learning of the autoencoder 108, the material database corresponding to the chemical space selected by the user is used instead of an arbitrary material database.

It is considered that the purpose of the user is to search a material having high material physical properties A. At this time, ideally, it is considered that there are a group of feature quantities most useful for describing a material set having high material physical properties A, and it is most desirable to perform machine learning using the feature quantities in order to increase a prediction accuracy.

It is considered that there is a material database X collected in another case in the past, and at this time the purpose of use is to search a material having material physical properties B. Here, in a case where it is found that there is a correlation between the material physical properties A and B by expert knowledge, the material database X is likely to overlap with the material set having high material physical properties A. Accordingly, it is considered that the material having high material physical properties A is easily searched by learning the autoencoder using the material database X and extracting the material feature quantities.

Therefore, in the description of a specific usage example in this example, as illustrated in Fig. 12(a), a material database X1201 that is one of the case data is extracted from the case-by-case material database 107, and the autoencoder 108 for obtaining feature quantities 1203 is learned by a structural formula 1202 thereof.

In the case-by-case material database 107, a caption in a text format or a tag may be applied to each of the case data items such that the user is capable of displaying or searching case the purpose or theme of the data, the main component of the material, the material properties that are stored, or the like. In addition, the purpose of use, the user, and other information items may be stored as past usage history of the case data to be capable of being displayed and searched. In the autoencoder 108, for example, a recurrent neural network (RNN) or a deep neural network (DNN) is used.

Fig. 12(a) illustrates an example in which the user who is examining the material physical properties A extracts the material database X1201 that is data obtained by examining the material physical properties B in the past, from the case-by-case material database 107. The user having professional knowledge finds that there is a relationship between the material physical properties A and B, and extracts the material database X1201 using the search function described above. The material database X1201, for example, includes the structural formula and the data of the material physical properties B. In the learning of the autoencoder 108, the structural formula 1202 is used.

As described above, the autoencoder 108 for obtaining suitable feature quantities from the structural formula is prepared, and then, a prediction model for assuming the material physical properties A is learned. As illustrated in Fig. 12(b), the structural formula and the data including the measured material physical properties A are prepared as training data 1204. The training data 1204 may be selected from the case-by-case material database 107, or new data may be acquired from other than the case-by-case material database 107.

A structural formula 1205 is acquired from the training data 1204 and input to the autoencoder 108 to obtain feature quantities 1206. Then, a prediction model 1208 is learned by using a set of the feature quantities 1206 and data 1207 of the material physical properties A as the training data. The data of the material physical properties A may be converted to suitable feature quantities. In the prediction model 1208, for example, RNN or DNN may be used, and in the learning, known supervised learning may be used.

Note that, in a case where the training data 1204 includes data other than the structural formula and the data of the material physical properties A (for example, a production condition of the material), the data may be added to the training data. As described above, the prediction model 1208 for assuming the material physical properties A is capable of being learned. The prediction model 1208 is implemented on the material property prediction unit 109.

After that, as illustrated in Fig. 12(c), the user prepares a structural formula 1209 of a material having the material physical properties A to be assumed. The structural formula 1209 is input from the material property prediction receiving unit 105. The structural formula is input to the autoencoder 108 to obtain feature quantities 1210. The feature quantities 1210 is input to the prediction model 1208 to obtain material physical properties A1211 that are assumed.

As described above, the feature quantities are generated by utilizing the material database collected in the past case associated with the purpose of the current material analysis, instead of constraint-free general open data or the like, and thus, accurate assumption can be performed.

In this example, in both of the learning of the autoencoder 108 and the learning of the prediction model in the material property prediction unit 109, the same case-by-case material database 107 is used, which does not limit the use of different databases. That is, a material database A and a material database B are prepared, and in the learning of the autoencoder 108, the material database A may be used, and in the learning of the prediction model, the material database B may be used. In this case, in the material database B, data of a material property value for each compound is required, but in the material database A, such data is not required. In addition, in the learning of the autoencoder 108, both of the material databases A and B may be used, and in the learning of the prediction model, only the material database B may be used.

The autoencoder 108 or the prediction model generated in Example may be stored in the storage device with text data for describing the content. As described above, the past model is processed into a library, and thus, can be reused as necessary.

As described above, since the user selects the chemical space from the case-by-case material database collecting the material data associated with the analysis purpose, and the autoencoder is learned, effective material feature quantities more coincident with the analysis purpose are generated, and thus, accurate prediction of the material properties is facilitated.

Example has been described, but the present invention is not limited to Example described above and includes various modification examples. For example, Example described above has been described in detail in order to facilitate the understanding of the present invention, and is not necessarily limited to include all configurations described above. In addition, it is possible to replace a part of the configuration of one Example with the configuration of another Example, and it is also possible to add the configuration of another Example to the configuration of one Example. In addition, it is possible to add, delete, and replace a part of the configuration of each Example with another configuration.

### REFERENCE SIGNS LIST

- 101: Material property prediction device
- 103: Chemical space designation unit
- 104: Autoencoder learning unit
- 105: Material property prediction receiving unit
- 106: Experimental data receiving unit
- 107: Case-by-case material database
- 108: Autoencoder
- 109: Material property prediction unit

## Claims

1. A material property prediction device for predicting a material property using a case-by-case material database storing a plurality of case databases,
the case database including a plurality of records recording structural information about material structures in association with material properties about properties of materials, the device comprising:
a chemical space designation unit receiving a designation of at least one case database;
an autoencoder learning unit generating an autoencoder for converting structural information corresponding to the case database received by the chemical space designation unit to multi-variables; and
a material property prediction unit predicting material properties using the multi-variables converted by the autoencoder generated by the autoencoder learning unit.

2. The material property prediction device according to claim 1,
wherein the autoencoder is a model having a property of enabling the structural information to be restored from the multi-variables after converting the structural information to the multi-variables.

3. The material property prediction device according to claim 1,
wherein the material property prediction unit
inputs training data including the plurality of records recording the structural information about the material structures in association with the material properties about the properties of the materials,
inputs structural information corresponding to the training data to the autoencoder and converts the structural information to multi-variables as explanatory variables, and
sets material properties corresponding to the training data as objective variables and trains a prediction model using the explanatory variables and the objective variables.

4. The material property prediction device according to claim 3, further comprising:
a material property prediction receiving unit receiving structural information about structures of materials having properties to be predicted,
wherein the material property prediction unit
inputs the structural information about the structures of the materials having the properties to be predicted to the autoencoder and converts the structural information to multi-variables as explanatory variables, and
inputs the explanatory variables to the prediction model and predicts properties that are the objective variables.

5. The material property prediction device according to claim 1,
wherein the chemical space designation unit has a function of searching the case database with a keyword.

6. A material property prediction method, executing:
a first step of preparing a first database including a plurality of records recording structural information about material structures;
a second step of extracting structural information from the first database prepared in the first step;
a third step of training an autoencoder for converting structural information to multi-variables using the structural information extracted in the second step;
a fourth step of preparing a second database including a plurality of records recording structural information about material structures in association with material properties about properties of materials;
a fifth step of extracting structural information from the second database prepared in the fourth step;
a sixth step of converting the structural information extracted in the fifth step to multi-variables using the autoencoder;
a seventh step of obtaining explanatory variables on the basis of the multi-variables converted in the sixth step and obtaining objective variables on the basis of material properties extracted from the second database; and
an eighth step of generating a prediction model for assuming the objective variables from the explanatory variables using the explanatory variables and the objective variables.

7. The material property prediction method according to claim 6,
wherein in the first step,
a case-by-case material database storing a plurality of case databases is used, and at least one case database is selected from the case-by-case material database as the first database.

8. The material property prediction method according to claim 7,
wherein in the case-by-case material database, text information is stored in association with the case database, and
in the first step,
a user searches the text information and selects at least one case database.

9. The material property prediction method according to claim 6,
wherein in the first step,
a case-by-case material database storing a plurality of case databases is used, and the case database includes a plurality of records recording structural information about material structures in association with material properties about properties of materials,
in the first step,
at least one case database is selected from the case-by-case material database as the first database, and
in the fourth step,
at least one case database is selected from the case-by-case material database as the second database.

10. The material property prediction method according to claim 9,
wherein the material properties included in the records of the first database and the material properties included in the records of the second database are material properties having different definitions.

11. The material property prediction method according to claim 6,
wherein in the autoencoder,
a model having a property of enabling the structural information to be restored from the multi-variables after converting the structural information to the multi-variables is used.

12. The material property prediction method according to claim 6, further executing:
a ninth step of preparing structural information about material structures having properties to be predicted;
a tenth step of converting the structural information prepared in the ninth step to multi-variables using the autoencoder;
an eleventh step of obtaining explanatory variables on the basis of the multi-variables converted in the tenth step; and
a twelfth step of assuming material properties that are the objective variables by applying the explanatory variables obtained in the eleventh step to the prediction model.

13. The material property prediction method according to claim 6,
wherein at least one of the autoencoder and the prediction model is stored in a storage device and reused.

14. The material property prediction method according to claim 6,
wherein both of the first database and the second database include the plurality of records recording the structural information about the material structures in association with the material properties about the properties of the materials, and record data having different definitions or types with respect to the material properties.
